# EUROPEAN PATENT APPLICATION

(11) **EP 4 438 964 A1**
(43) Date of publication of application: **02.10.2024**
(21) Application number: 24161469.2
(22) Date of filing: 05.03.2024
(51) Int. Cl.: F24F 8/10, F24F 11/00, F24F 110/50, F24F 110/64, F24F 110/65, F24F 110/66, F24F 110/70, F24F 110/72, F24F 110/74, F24F 11/30

(54) **SYSTEM FOR CLEANING, ELIMINATING, AND PREVENTING AIR POLLUTION IN AN INDOOR SPACE**

(30) Priority: 29.03.2023 TW 112112047
(71) Applicant: Microjet Technology Co., Ltd., Hsinchu (TW)
(72) Inventor: Mou, Hao-Jan, Hsinchu (TW); Wu, Chin-Chuan, Hsinchu (TW); Han, Yung-Lung, Hsinchu (TW); Huang, Chi-Feng, Hsinchu (TW)
(74) Representative: Uexküll & Stolberg

(57) **Abstract**

A system for cleaning, zeroing, and preventing air pollution in indoor space includes several gas detection devices (A, 3), a central control monitoring device (B), and several gas exchange and filtration devices (C). The gas detection devices (A, 3) detect and output indoor and outdoor air pollution data. The central control monitoring device (B) receives the outdoor and indoor air pollution data to perform an intelligent computation and comparison, locate an air pollution location in the indoor space, and transmit a control command intelligently. Each of the gas exchange and filtration devices (C) is combined with a smart switch (D) for the control command to control the gas exchange and filtration devices (C) to be enabled, so that the air pollution is filtered and exchanged by the gas exchange and filtration devices (C), thereby allowing the indoor air pollution data detected by the gas detection devices (A, 3) in the indoor space to be a safety detection value.

## Description

### BACKGROUND

### Technical Field

The present invention relates to a system for cleaning, zeroing, and preventing air pollution in indoor space, in particular, to a system adapted to be implemented in an indoor space, the system including: a plurality of gas detection devices, a central control monitoring device, and a plurality of gas exchange and filtration devices are utilized to implement the detection, the filtration, and gas-exchange of the air pollution to allow the indoor air pollution data to approach to a non-detection state (almost zero) or equal to zero, making the gas (air) of the indoor space to a safe and breathable state.

### Related Art

In light of people paying more and more attention to the ambient gas quality in daily life, it is noted that the particulate matters (PM1, PM2.5, PM10), carbon dioxide, total volatile organic compounds (TVOC), formaldehyde and even particulates, aerogels, bacteria, viruses contained in the air might affect the human health, even might be life-threatening when exposure to these gases. It is understood that, currently, it is not easy to control the indoor air quality since the affecting factors of the indoor air quality include not only the outdoor space air quality but also the air conditioning and the pollution sources in the indoor space (especially the dusts originated from poor circulation of air in the indoor space). Therefore, air conditioners or air cleaners are utilized for improving the indoor air quality.

Consequently, it is an issue of the present invention for intelligently and rapidly detecting the indoor air pollution source thereby effectively removing the air pollution from the indoor space, monitoring the air quality of the indoor space whenever and wherever possible, making the air into a safe and breathable state while the air quality in the indoor space is lowering than a default standard, and generating an air convection intelligently to detect and locate the air pollution rapidly and to effectively control a plurality of gas exchange and filtration devices to perform an intelligent air convection to allow the gas (air) in the indoor space to be cleaned to a safe and breathable state.

### SUMMARY

One object of the present invention is to provide a system for cleaning, zeroing, and preventing air pollution in indoor space. The system including: a plurality of gas detection devices is utilized to monitor the gas quality in the environment. The gas detection devices transmit signal(s) to a central control monitoring device to perform intelligent computation and comparison, and the central control monitoring device is connected to a cloud device to perform various mathematical computation and artificial intelligent computation to ensure the air pollution location. The central control monitoring device selectively and intelligently transmits a control command to enable a gas exchange and filtration device closest to the air pollution location to generate an air flow to guide the air pollution to be filtered by at least one gas exchange and filtration device. The central control monitoring device controls the gas exchange and filtration devices to be enabled according to the gas quality of the environment detected by the system, so that the air pollution is filtered and exchanged by the gas exchange and filtration devices, thereby allowing the indoor air pollution data detected by the gas detection devices in the indoor space to be a safety detection value in which the indoor air pollution data approaches to a non-detection state or equals to zero, and the gas in the indoor space is cleaned to a safe and breathable state.

In order to accomplish the above object(s), in the general embodiment of the present invention, a system for cleaning, zeroing, and preventing air pollution in indoor space is adapted to be implemented in an indoor space, the system comprises a plurality of gas detection devices, a central control monitoring device, and a plurality of gas exchange and filtration devices. The gas detection devices are configured to detect an air pollution in the indoor space. The gas detection devices comprise at least one outdoor gas detection device and at least one indoor gas detection device. The at least outdoor gas detection device and the at least one indoor gas detection device are configured to detect a qualitative property and a concentration of the air pollution and respectively output an outdoor air pollution data and an indoor air pollution data. The central control monitoring device is configured to receive the outdoor air pollution data and the indoor air pollution data through a wireless communication to perform an intelligent computation and comparison on the outdoor air pollution data and the indoor air pollution data to locate an air pollution location in the indoor space, and the central control monitoring device is configured to transmit a control command intelligently and selectively through the wireless communication. Each of the gas exchange and filtration devices is combined with a smart switch, and the smart switch receives the control command to perform an adjusting and controlling mechanism, so that the air pollution is filtered and exchanged by the gas exchange and filtration devices, thereby allowing the indoor air pollution data detected by the gas detection devices in the indoor space to be a safety detection value in which the indoor air pollution data approaches to a non-detection state or equals to zero, and the gas in the indoor space is cleaned to a safe and breathable state.

### BRIEF DESCRIPTION OF THE DRAWINGS

The invention will become more fully understood from the detailed description given herein below, the illustration is only for describing and thus not limitative of the invention, wherein:
Fig. 1A illustrates a schematic view showing the operation of a system for cleaning, zeroing, and preventing air pollution in indoor space of an embodiment in the present invention, wherein the system is utilized in an indoor space;
Fig. 1B illustrates a schematic view showing the operation of a system for cleaning, zeroing, and preventing air pollution in indoor space of another embodiment in the present invention, wherein the system is utilized in the indoor space;
Fig. 2A illustrates a schematic view of a gas detection device including an external power terminal of an exemplary embodiment in the present invention;
Fig. 2B illustrates a schematic sectional view showing the assembling relationship among components of the gas detection device including the external power terminal of the exemplary embodiment in the present invention;
Fig. 2C illustrates a schematic sectional view showing the assembling relationship among components of a gas detection device not including the external power terminal of the exemplary embodiment in the present invention;
Fig. 3A illustrates a perspective view showing the assembling relationship among components of a gas exchange and filtration device of the exemplary embodiment in the present invention;
Fig. 3B illustrates a schematic view of a filtering component of the gas exchange and filtration device of the exemplary embodiment in the present invention;
Fig. 4A illustrates an assembled schematic view (1) of a gas detection main body of the gas detection device of the exemplary embodiment in the present invention;
Fig. 4B illustrates an assembled schematic view (2) of the gas detection main body of the gas detection device of the exemplary embodiment in the present invention;
Fig. 4C illustrates an exploded view of the gas detection main body of the gas detection device of the exemplary embodiment in the present invention;
Fig. 5A illustrates a perspective view (1) of a base of the gas detection main body of the exemplary embodiment in the present invention;
Fig. 5B illustrates a perspective view (2) of the gas detection main body of the exemplary embodiment in the present invention;
Fig. 6 illustrates a perspective view (3) of the base of the gas detection main body of the exemplary embodiment in the present invention;
Fig. 7A illustrates an exploded view of a piezoelectric actuator separating from the base of the gas detection main body of the exemplary embodiment in the present invention;
Fig. 7B illustrates a perspective view of the base in combination with the piezoelectric actuator of the gas detection main body of the exemplary embodiment in the present invention;
Fig. 8A illustrates an exploded view (1) of the piezoelectric actuator of the exemplary embodiment in the present invention;
Fig. 8B illustrates an exploded view (2) of the piezoelectric actuator of of the exemplary embodiment in the present invention;
Fig. 9A illustrates a cross-sectional view (1) showing the operation of the piezoelectric actuator of the exemplary embodiment in the present invention;
Fig. 9B illustrates a cross-sectional view (2) showing the operation of the piezoelectric actuator of the exemplary embodiment in the present invention;
Fig. 9C illustrates a cross-sectional view showing the operation (3) of the piezoelectric actuator of the exemplary embodiment in the present invention;
Fig. 10A illustrates a cross-sectional view (1) of the gas detection main body of the exemplary embodiment in the present invention;
Fig. 10B illustrates a cross-sectional view (2) of the gas detection main body of the exemplary embodiment in the present invention;
Fig. 10C illustrates a cross-sectional view (3) of the gas detection main body of the exemplary embodiment in the present invention; and
Fig. 11 illustrates a schematic view showing the transmission of the gas detection device of the exemplary embodiment in the present invention.

### DETAILED DESCRIPTION

The present invention will now be described more specifically with reference to the following embodiments. It is to be noted that the following descriptions of different embodiments of this invention are presented herein for purpose of illustration and description only, and it is not intended to limit the scope of the present invention.

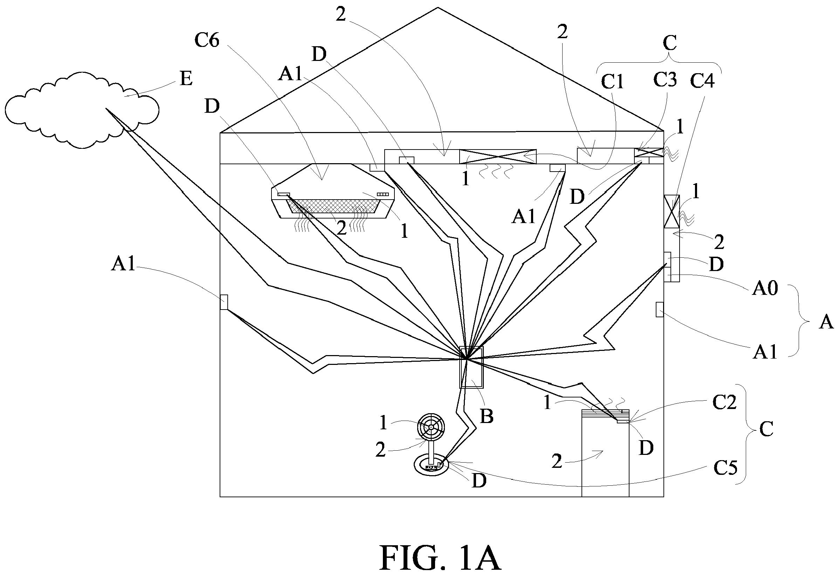

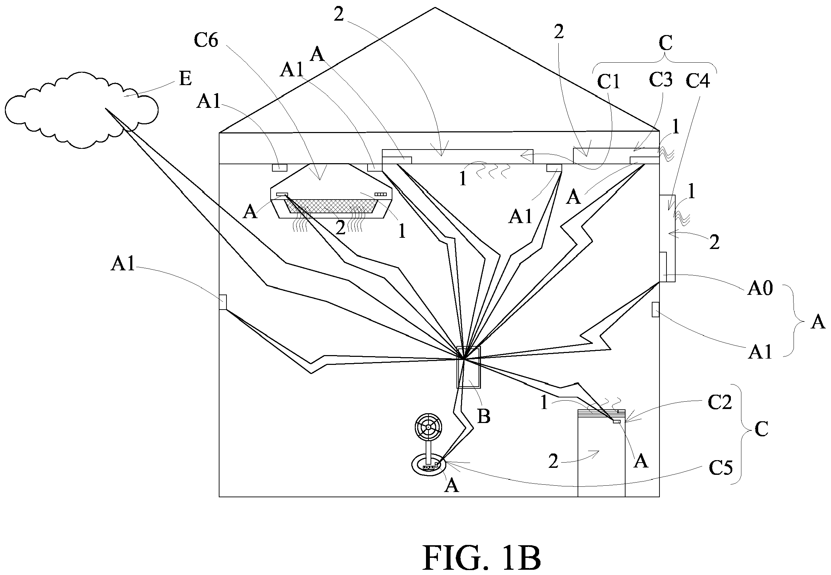

Please refer to Fig. 1A and Fig. 1B, according to one or some embodiments of the present invention, a system for cleaning, zeroing, and preventing air pollution in indoor space is adapted to be implemented in an indoor space, and the system comprises a plurality of gas detection devices A, a central control monitoring device B, and a plurality of gas exchange and filtration devices C. The gas detection devices A are configured to detect a qualitative property and a concentration of the air pollution and output an outdoor air pollution data and an indoor air pollution data. The central control monitoring device B is configured to receive the outdoor air pollution data and the indoor air pollution data to perform an intelligent computation and comparison on the outdoor air pollution data and the indoor air pollution data to locate an air pollution location in the indoor space, and the central control monitoring device B is configured to transmit a control command intelligently and selectively through a wireless communication. The gas exchange and filtration devices C are configured to receive the control command to be enabled, so that the air pollution is filtered and exchanged by the gas exchange and filtration devices C, until the indoor air pollution data detected by the gas detection devices A in the indoor space become a safety detection value, thus the air pollution in the indoor space is cleaned and the indoor air pollution data approaches to a non-detection state or equals to zero, and the gas in the indoor space is cleaned to a safe and breathable state. For further definition of the air pollution (namely the polluted gas or polluted air) as mentioned in the above of embodiments, the air pollution may include at least one selected from the group consisting of particulate matters, carbon monoxide (CO), carbon dioxide (CO₂), ozone (O₃), sulfur dioxide (SO₂), nitrogen dioxide (NO₂), lead (Pb), total volatile organic compounds (TVOC), formaldehyde (HCHO), bacteria, fungi, viruses, and any combination thereof.

The gas detection devices A comprise at least one outdoor gas detection device A0 and at least one indoor gas detection device A1. The at least one outdoor gas detection device A0 is configured to detect a qualitative property and a concentration of an air pollution of an outdoor gas and transmit an outdoor air pollution data, the at least one indoor gas detection device A1 is configured to detect a qualitative property and a concentration of an air pollution in the indoor space and output an indoor air pollution data. In some embodiments, the transmission of the outdoor air pollution data and the indoor air pollution data is implemented through a wireless communication, and the wireless communication is implemented by using one of a Wi-Fi module, a Bluetooth module, a radiofrequency identification module, and a near field communication module.

The central control monitoring device B is configured to receive the outdoor air pollution data and the indoor air pollution data through the wireless communication to perform an intelligent computation and comparison on the outdoor air pollution data and the indoor air pollution data to locate an air pollution location in the indoor space, and the central control monitoring device B is configured to transmit a control command intelligently and selectively. In some embodiments, the wireless communication is implemented by using one of a Wi-Fi module, a Bluetooth module, a radiofrequency identification module, and a near field communication module.

Each of the gas exchange and filtration devices C comprises at least one blower 1 and at least one filtrating component 2, and each of the gas exchange and filtration devices C is combined with a smart switch D. The smart switch D receives the control command to control the at least one blower 1 to be enabled, so that the air pollution is filtered and exchanged by the filtering component 2, until the indoor air pollution data detected by the gas detection devices A in the indoor space become the safety detection value, thus the air pollution in the indoor space is cleaned and the indoor air pollution data approaches to the non-detection state or equals to zero, and the gas in the indoor space is cleaned to the safe and breathable state. In some embodiments, the safety detection value includes at least one selected from the group consisting of a concentration of PM2.5 which is less than 15 µg/m³, a concentration of carbon dioxide which is less than 1000 ppm, a concentration of total volatile organic compounds which is less than 0.56 ppm, a concentration of formaldehyde which is less than 0.08 ppm, a colony-forming unit per cubic meter of bacteria which is less than 1500 CFU/m³, a colony-forming unit per cubic meter of fungi which is less than 1000 CFU/m³, a concentration of sulfur dioxide which is less than 0.075 ppm, a concentration of nitrogen dioxide which is less than 0.1 ppm, a concentration of carbon monoxide which is less than 9 ppm, a concentration of ozone which is less than 0.06 ppm, a concentration of lead which is less than 0.15 µg/m³, and any combination thereof.

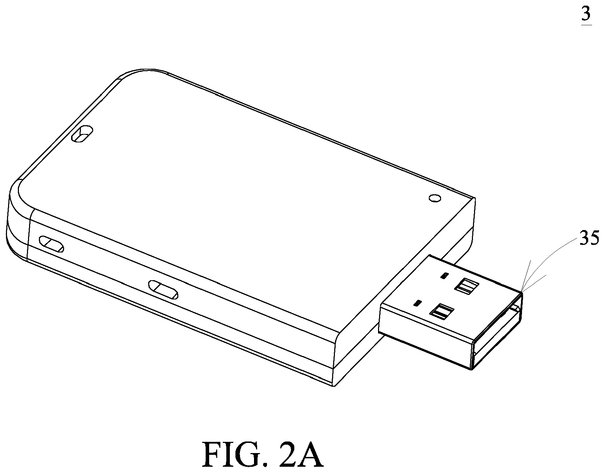

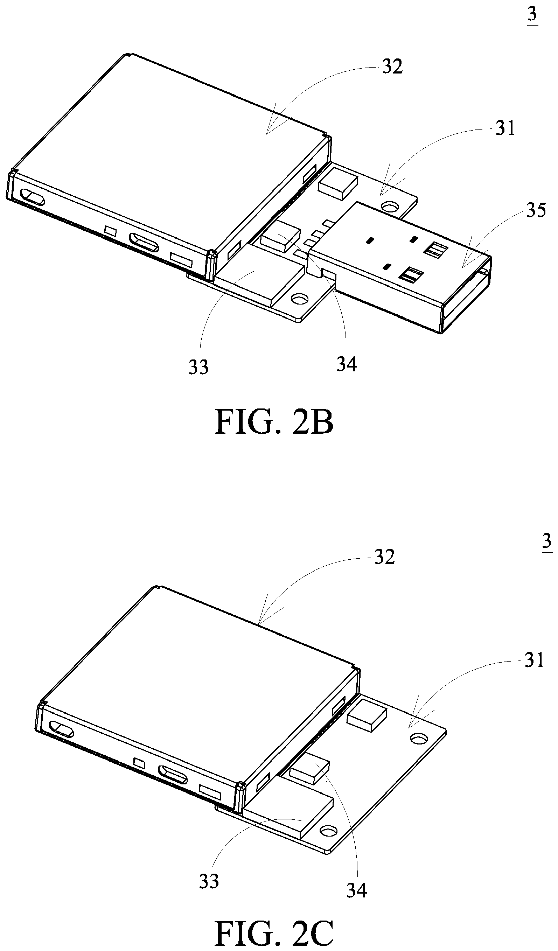

The outdoor gas detection device A0 and the indoor gas detection device A1 may be, but not limited to, a gas detection device having an identical structure, which would be indicated by reference number 3 in the descriptions below. The gas detection device 3 may be of a configuration shown in Fig. 2A and Fig. 2B, in which the gas detection device 3 includes an external power terminal 35, the external power terminal 35 is integrated with a control circuit board 31 and protrudes out of the housing of the gas detection device 3. In another embodiment, the gas detection device 3 may be of a configuration shown in 2C, in which the gas detection device 3 do not include the external power terminal.

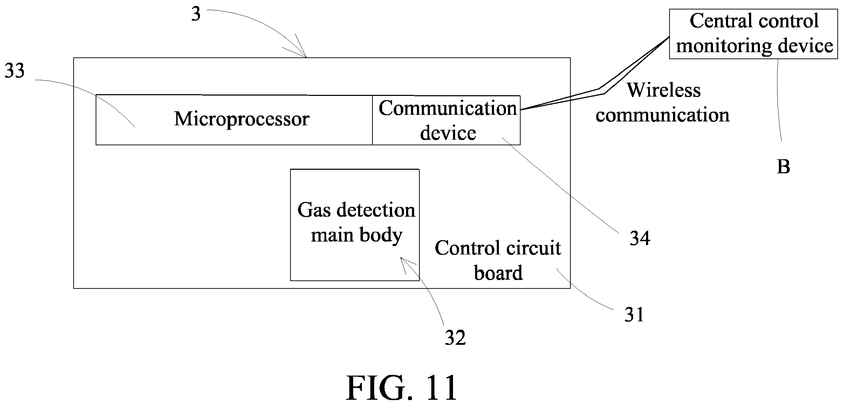

The gas detection device 3 includes a control circuit board 31, a gas detection main body 32, a microprocessor 33, and a communication device 34. The gas detection main body 32, the microprocessor 33, and the communication device 34 are integrally packaged with the control circuit board 31 and electrically connected to each other, and the microprocessor 33 controls the gas detection main body 32 to enable the operation of the gas detection main body 32, so that the gas detection main body 32 detects the air pollution and outputs a detection signal, and the microprocessor 33 receives the detection signal so as to compute, process, and output the air pollution data, therefore the microprocessor 33 provides the communication device 34 with the air pollution data through the wireless communication. In this embodiment, the communication device 34 outputs the indoor air pollution data or the outdoor air pollution data to the central control monitoring device B through the wireless communication (as shown in Fig. 11). The communication device 34 outputs the indoor air pollution data or the outdoor air pollution data through the wireless communication, and the wireless communication may be implemented by using one of a Wi-Fi module, a Bluetooth module, a radiofrequency identification module, and a near field communication module.

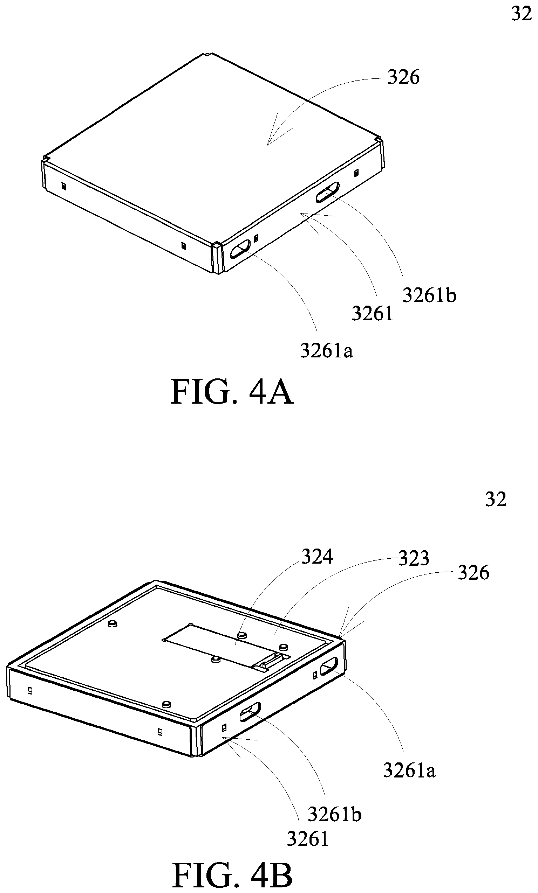

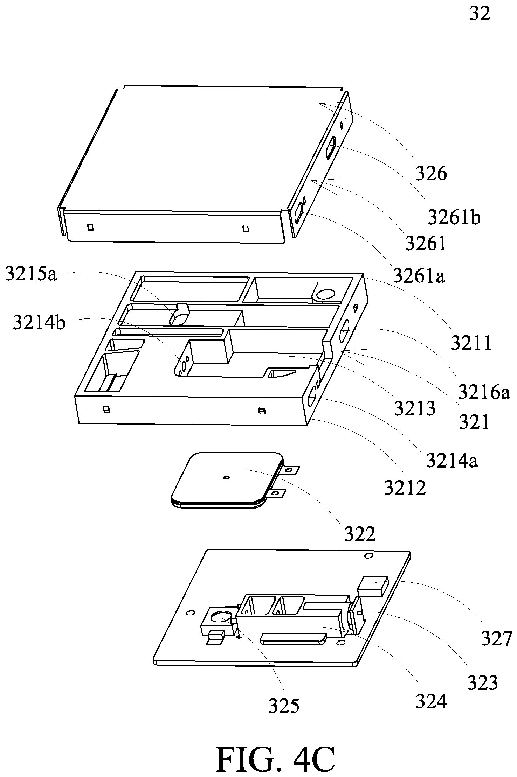

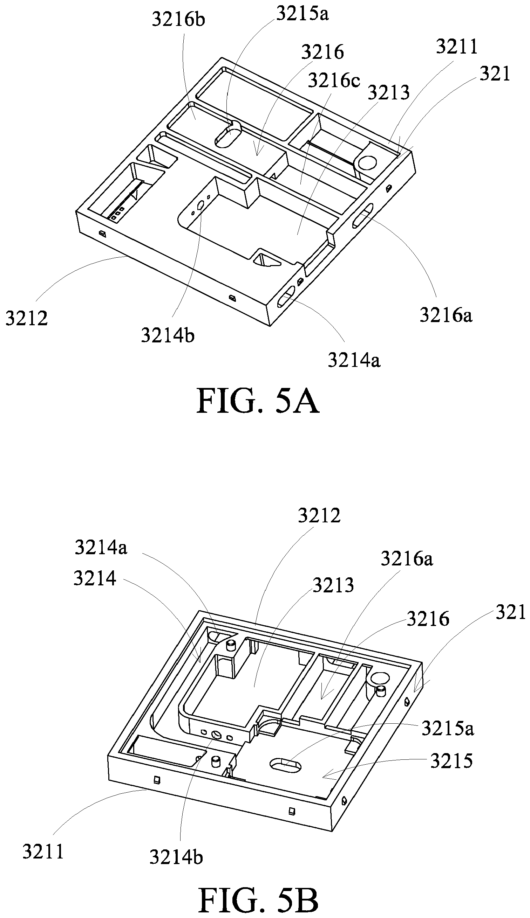

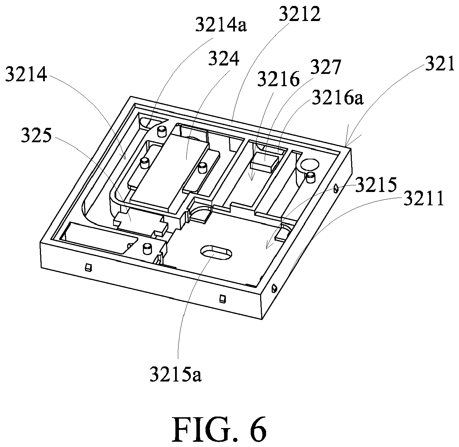

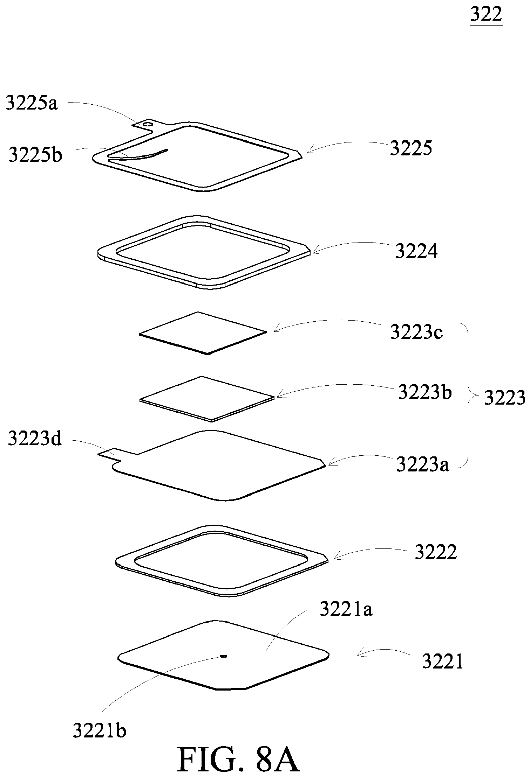

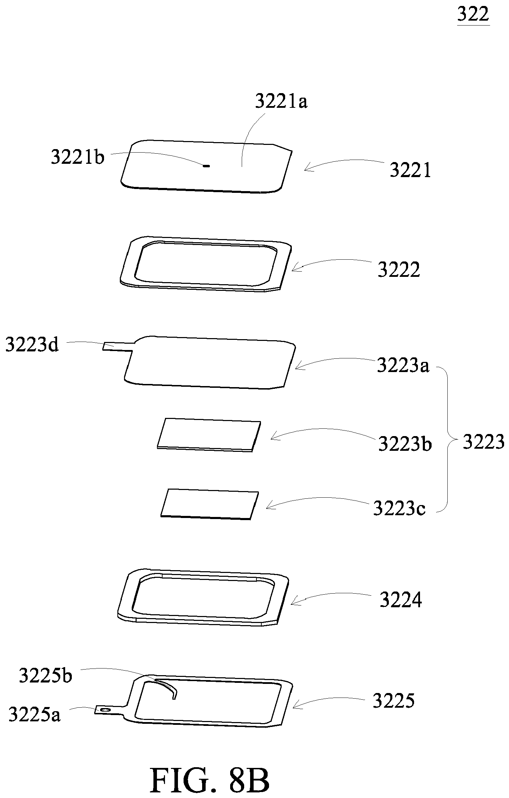

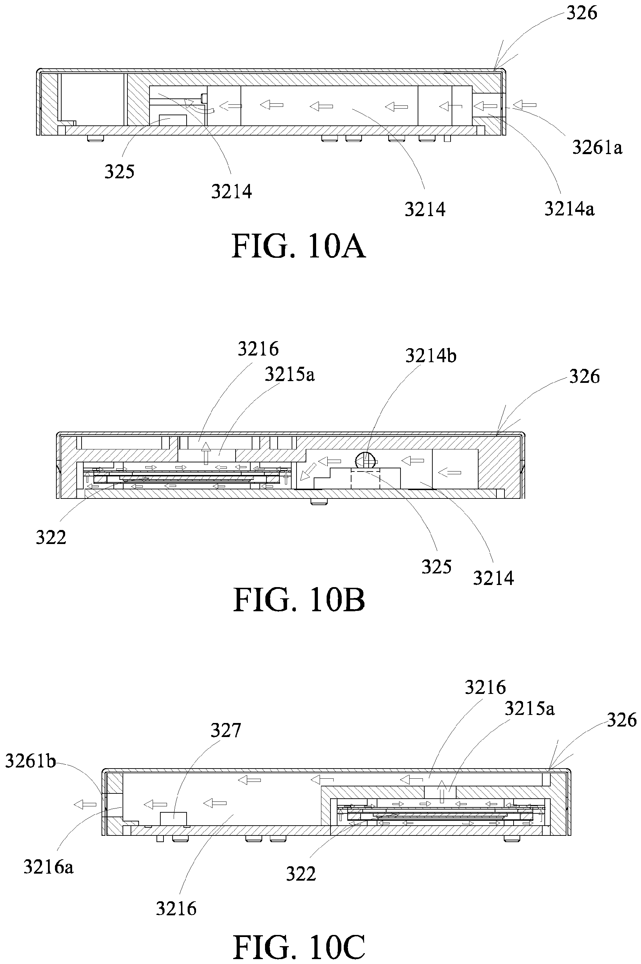

Please refer to Fig. 4A to Fig. 10C. In one or some embodiments, the gas detection main body 32 includes a base 321, a piezoelectric actuator 322, a driving circuit board 323, a laser component 324, a particulate sensor 325, and an outer cover 326. The base 321 has a first surface 3211, a second surface 3212, a laser installation region 3213, a gas inlet groove 3214, a gas-guiding component installation region 3215, and a gas outlet groove 3216. The first surface 3211 and the second surface 3212 are opposite to each other. The laser installation region 3213 is formed by hollowing out the base 321 from the first surface 3211 to the second surface 3212 for accommodating the laser component 324. The outer cover 326 covers the base 321 and has a side plate 3261. The side plate 3261 has a gas inlet opening 3261a and a gas outlet opening 3261b. The gas inlet groove 3214 is recessed from the second surface 3212 and located adjacent to the laser installation region 3213. The gas inlet groove 3214 has a gas inlet through hole 3214a and two lateral walls. The gas inlet through hole 3214a is in communication with the outside environment of the base 321 and is corresponding to the gas inlet opening 3261a of the outer cover 326. Two light penetration windows 3214b penetrate the two lateral walls of the gas inlet groove 3214 and are in communication with the laser installation region 3213. Therefore, when the first surface 3211 of the base 321 is covered by the outer cover 326, and the second surface 3212 of the base 321 is covered by the driving circuit board 323, a gas inlet path can be defined by the gas inlet groove 3214.

The gas-guiding component installation region 3215 is recessed from the second surface 3212 and in communication with the gas inlet groove 3214. A ventilation hole 3215a penetrates a bottom surface of the gas-guiding component installation region 3215. Each of the four corners of the gas-guiding component installation region 3215 has a positioning bump 3215b. The gas outlet groove 3216 has a gas outlet through hole 3216a, and the gas outlet through hole 3216a is corresponding to the gas outlet opening 3261b of the outer cover 326. The gas outlet groove 3216 includes a first region 3216b and a second region 3216c. The first region 3216b is recessed from a portion of the first surface 3211 corresponding to a vertical projection region of the gas-guiding component installation region 3215. The second region 3216c is at a portion extending from a region that is not corresponding to the vertical projection region of the gas-guiding component installation region 3215, and the second region 3216c is hollowed out from the first surface 3211 to the second surface 3212. The first region 3216b is connected to the second region 3216c to form a stepped structure. Moreover, the first region 3216b of the gas outlet groove 3216 is in communication with the ventilation hole 3215a of the gas-guiding component installation region 3215, and the second region 3216c of the gas outlet groove 3216 is in communication with the gas outlet through hole 3216a. Therefore, when the first surface 3211 of the base 321 is covered by the outer cover 326 and the second surface 3212 of the base 321 is covered by the driving circuit board 323, a gas outlet path can be defined by the gas outlet groove 3216 and the driving circuit board 323.

Furthermore, the laser component 324 and the particulate sensor 325 are disposed on the driving circuit board 323 and located in the base 321. The laser component 324 and the particulate sensor 325 are electrically connected to the driving circuit board 323. It should notice that the driving circuit board 323 is omitted to clearly explain the positions of the laser component 324, the particulate sensor 325, and the base 321. In the embodiment of the present invention, the laser component 324 is located at the laser installation region 3213 of the base 321. The particulate sensor 325 is located at the gas inlet groove 3214 of the base 321 and aligned with the laser component 324. Moreover, the laser component 324 is corresponding to the light penetration windows 3214b so as to allow the light beam emitted by the laser component 324 to pass therethrough and into the gas inlet groove 3214. The light path of the light beam emitted by the laser component 324 passes through the light penetration windows 3214b and is orthogonal to the gas inlet groove 3214. The light beam emitted by the laser component 324 passes into the gas inlet groove 3214 through the light penetration windows 3214b, thereby the particulate matters in the gas inlet groove 3214 is illuminated by the light beam. When the light beam contacts the gas, the light beam will be scattered and generate light spots. Hence, the light spots generated by the scattering are received and calculated by the particulate sensor 325 located at the position orthogonal to the gas inlet groove 3214 to obtain the detection data of the gas. Furthermore, a gas sensor 327 is disposed on the driving circuit board 323 and is located at the gas outlet groove 3216 for detecting the polluted gas introduced into the gas outlet groove 3216, and the gas sensor 327 is electrically connected to the driving circuit board 323. In one embodiment of the present invention, the gas sensor 327 includes at least one selected from the group consisting of a volatile organic compound detector capable of detecting gas information of carbon dioxide (CO₂) or total volatile organic compounds (TVOC), a formaldehyde sensor capable of detecting gas information of formaldehyde (HCHO) gas, a bacterial sensor capable of detecting information of bacteria or fungi, and a virus sensor capable of detecting information of viruses, and any combination thereof.

Moreover, the piezoelectric actuator 322 is located at the square-shaped gas-guiding component installation region 3215 of the base 321, and the gas-guiding component installation region 3215 is in communication with the gas inlet groove 3214. When the piezoelectric actuator 322 is enabled, the gas in the gas inlet groove 3214 is inhaled into the piezoelectric actuator 322, passing through the ventilation hole 3215a of the gas-guiding component installation region 3215, and entering the gas outlet groove 3216. Moreover, the driving circuit board 323 covers the second surface 3212 of the base 321. The laser component 324 and the particulate sensor 325 are disposed on the driving circuit board 323 and electrically connected to the driving circuit board 323. As the outer cover 326 covers the base 321, the gas inlet opening 3261a is corresponding to the gas inlet through hole 3214a of the base 321, and the gas outlet opening 3216b is corresponding to the gas outlet through hole 3216a of the base 321.

Furthermore, the piezoelectric actuator 322 includes a nozzle plate 3221, a chamber frame 3222, an actuation body 3223, an insulation frame 3224, and a conductive frame 3225. The nozzle plate 3221 is made by a flexible material and has a suspension sheet 3221a and a hollow hole 3221b. The suspension sheet 3221a is a flexible sheet which can bend and vibrate. The shape and the size of the suspension sheet 3221a approximately corresponding to the inner edge of the gas-guiding component installation region 3215. The hollow hole 3221b penetrates through the center portion of the suspension sheet 3221a for the gas flowing therethrough. In one embodiment of the present invention, the shape of the suspension sheet 3221a can be selected from square, circle, ellipse, triangle, or polygon.

Furthermore, the chamber frame 3222 is stacked on the nozzle plate 3221, and the shape of the chamber frame 3222 is corresponding to the shape of the nozzle plate 3221. The actuation body 3223 is stacked on the chamber frame 3222. A resonance chamber 3226 is collectively defined between the actuation body 3223, the chamber frame 3222, and the suspension sheet 3221a. The insulation frame 3224 is stacked on the actuation body 3223. The appearance of the insulation frame 3224 is similar to the appearance of the chamber frame 3222. The conductive frame 3225 is stacked on the insulation frame 3224. The appearance of the conductive frame 3225 is similar to the appearance of the insulation frame 3224. The conductive frame 3225 has a conductive pin 3225a and a conductive electrode 3225b. The conductive pin 3225a extends outwardly from the outer edge of the conductive frame 3225, and the conductive electrode 3225b extends inwardly from the inner edge of the conductive frame 3225. Moreover, the actuation body 3223 further includes a piezoelectric carrying plate 3223a, an adjusting resonance plate 3223b, and a piezoelectric plate 3223c. The piezoelectric carrying plate 3223a is stacked on the chamber frame 3222, and the adjusting resonance plate 3223b is stacked on the piezoelectric carrying plate 3223a. The piezoelectric plate 3223c is stacked on the adjusting resonance plate 3223b. The adjusting resonance plate 3223b and the piezoelectric plate 3223c are accommodated in the insulation frame 3224. The conductive electrode 3225b of the conductive frame 3225 is electrically connected to the piezoelectric plate 3223c. In one preferred embodiment of the present invention, the piezoelectric carrying plate 3223a and the adjusting resonance plate 3223b are both made of conductive material(s). The piezoelectric carrying plate 3223a has a piezoelectric pin 3223d. The piezoelectric pin 3223d and the conductive pin 3225a are in electrical connection with a driving circuit (not shown) of the driving circuit board 323 to receive a driving signal (which may be a driving frequency and a driving voltage). The piezoelectric pin 3223d, the piezoelectric carrying plate 3223a, the adjusting resonance plate 3223b, the piezoelectric plate 3223c, the conductive electrode 3225b, the conductive frame 3225, and the conductive pin 3225a may together generate an electrical circuit for transmitting the driving signal, and the insulation frame 3224 is provided for electrically insulating the conductive frame 3225 from the actuation body 3223 to avoid short circuit, thereby the driving signal can be transmitted to the piezoelectric plate 3223c. When the piezoelectric plate 3223c receives the driving signal, the piezoelectric plate 3223c deforms owing to the piezoelectric effect, and thus the piezoelectric carrying plate 3223a and the adjusting resonance plate 3223b are driven to vibrate in a reciprocating manner.

Moreover, the adjusting resonance plate 3223b is disposed between the piezoelectric plate 3223c and the piezoelectric carrying plate 3223a as a buffering element so as to adjust the vibration frequency of the piezoelectric carrying plate 3223a. Generally, the thickness of the adjusting resonance plate 3223b is greater than the thickness of the piezoelectric carrying plate 3223a. The thickness of the adjusting resonance plate 3223b may be modified to adjust the vibration frequency of the actuation body 3223.

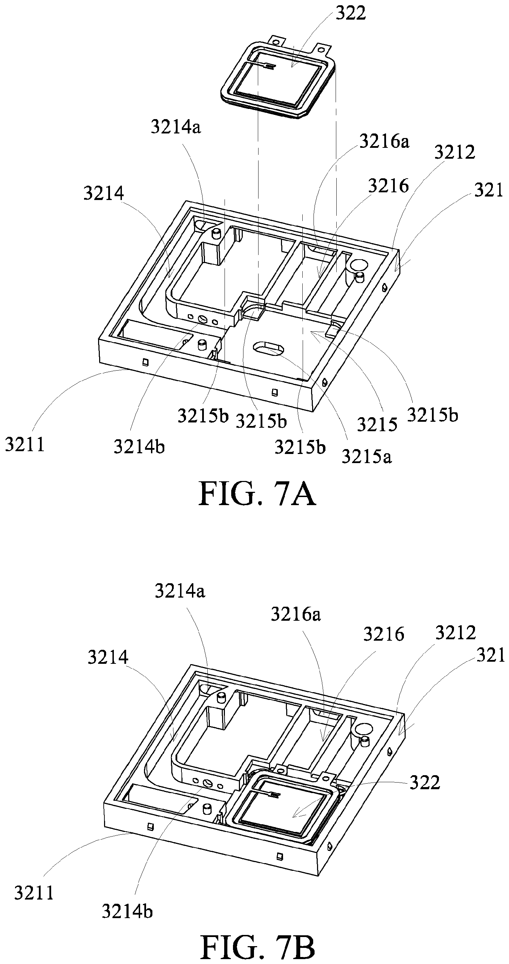

Please refer to Fig. 7A, Fig. 7B, Fig. 8A, Fig. 8B, and Fig. 9A. The nozzle plate 3221, the chamber frame 3222, the actuation body 3223, the insulation frame 3224, and the conductive frame 3225 are sequentially stacked and assembled and are positioned in the gas-guiding component installation region 3215, thereby a clearance 3221c is defined between the suspension sheet 3221a and the inner edge of the gas-guiding component installation region 3215 for the gas to pass therethrough. A gas flow chamber 3227 is formed between the nozzle plate 3221 and the bottom surface of the gas-guiding component installation region 3215. The gas flow chamber 3227 is in communication with the resonance chamber 3226 formed between the actuation body 3223, the chamber frame 3222, and the suspension sheet 3221a through the hollow hole 3221b of the nozzle plate 3221. In one aspect of the present invention, the resonance chamber 3226 and the suspension sheet 3221a can generate the Helmholtz resonance effect to improve the transmission efficiency of the gas through controlling the vibration frequency of the gas in the resonance chamber 3226 to be close to the vibration frequency of the suspension sheet 3221a. When the piezoelectric plate 3223c moves in a direction away from the bottom surface of the gas-guiding component installation region 3215, the piezoelectric plate 3223c drives the suspension sheet 3221a of the nozzle plate 3221 to move in the direction away from the bottom surface of the gas-guiding component installation region 3215 correspondingly. Hence, the volume of the gas flow chamber 3227 expands dramatically, therefore the internal pressure of the gas flow chamber 3227 decreases and creates a negative pressure, drawing the gas outside the piezoelectric actuator 322 to flow into the piezoelectric actuator 322 through the clearance 3221c and enter the resonance chamber 3226 through the hollow hole 3221b, thereby increasing the gas pressure of the resonance chamber 3226 and thus generating a pressure gradient. When the piezoelectric plate 3223c drives the suspension sheet 3221a of the nozzle plate 3221 to move toward the bottom surface of the gas-guiding component installation region 3215, the gas inside the resonance chamber 3226 is pushed to flow out rapidly through the hollow hole 3221b to further push the gas inside the gas flow chamber 3227, thereby the converged gas can be rapidly and massively ejected out of the gas flow chamber 3227 through the ventilation hole 3215a of the gas-guiding component installation region 3215 in a state closing to an ideal gas state under the Bernoulli's law.

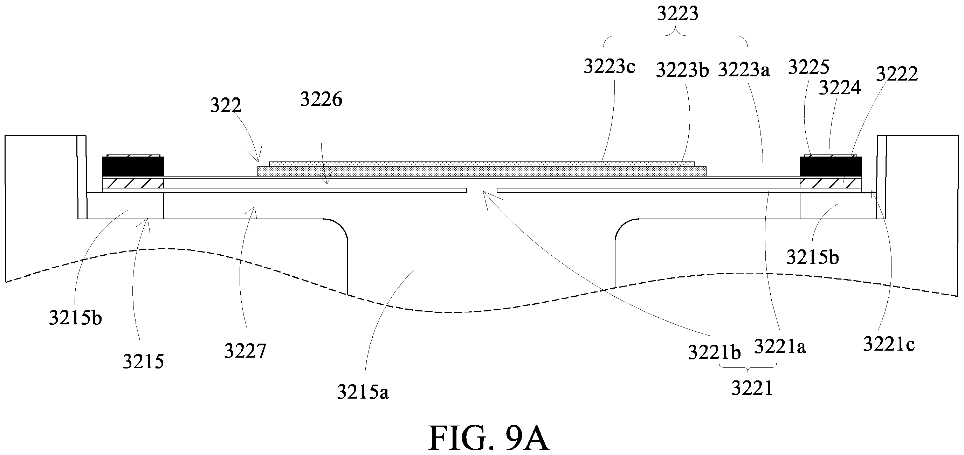

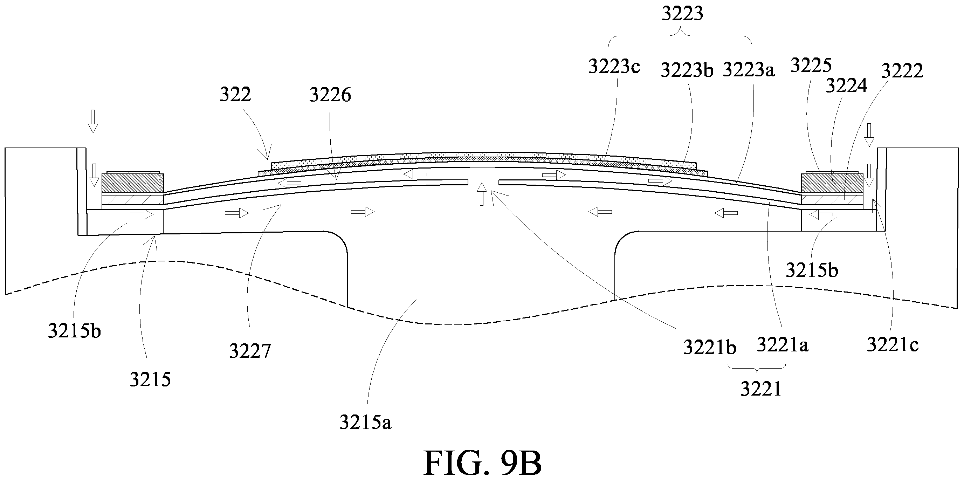

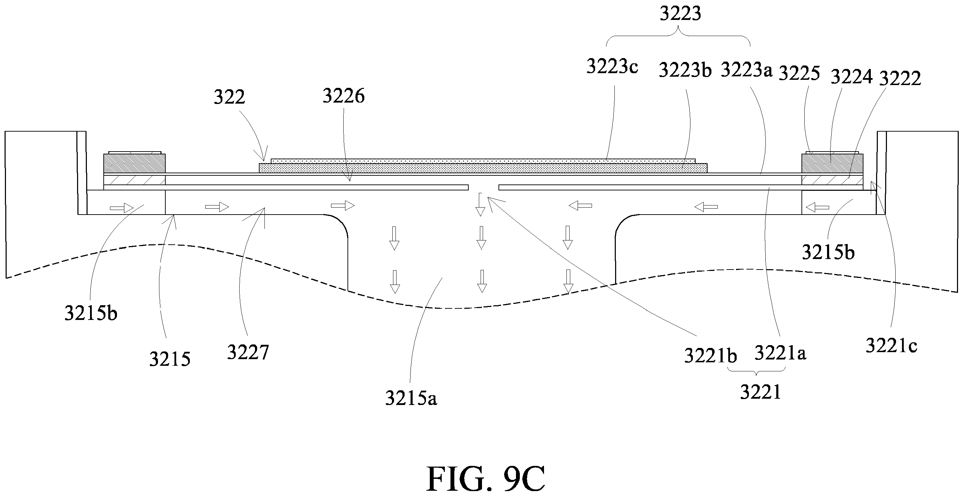

Therefore, through repeating the steps as shown in Fig. 9B and Fig. 9C, the piezoelectric plate 3223c can bend and vibrate in a reciprocating manner. Further, after the gas is discharged out of the resonance chamber 3226, the internal pressure of the resonance chamber 3226 is lower than the equilibrium pressure due to the inertia, as a result, the pressure difference guides the gas outside the resonance chamber 3226 into the resonance chamber 3226 again. Therefore, through controlling the vibration frequency of the gas in the resonance chamber 3226 to be close to the vibration frequency of the piezoelectric plate 3223c, the resonance chamber 3226 and the piezoelectric plate 3223c can generate the Helmholtz resonance effect so as to achieve effective, high-speed, and large-volume gas transmission of the gas. Moreover, the gas enters the gas detection main body 32 from the gas inlet opening 3261a of the outer cover 326, and flows into the gas inlet groove 3214 of the base 321 through the gas inlet through hole 3214a, eventually reaching the position of the particulate sensor 325. Furthermore, the piezoelectric actuator 322 continuously drives the gas into the gas inlet path so as to facilitate the gas inside the detection main body 32 to stably and rapidly pass through the particulate sensor 325. Next, the light beam emitted by the laser component 324 passes through the light penetration windows 3214b, enters the gas inlet groove 3214, and illuminates the gas in the gas inlet groove 3214 which passes through the particulate sensor 325. When the light beam from the particulate sensor 325 illuminates on the particulate matters in the gas, the light beam will be scattered and generate light spots. The particulate sensor 325 receives and calculates the light spots generated by the scattering to obtain the information of the particulate matters in the gas such as the particle size and the number of the particulate matters. Moreover, the gas passing through the particulate sensor 325 is continuously introduced into the ventilation hole 3215a of the gas-guiding component installation region 3215 by the piezoelectric actuator 322 and enters the gas outlet groove 3216. Finally, after the gas enters the gas outlet groove 3216, since the piezoelectric actuator 322 continuously delivers the gas into gas outlet groove 3216, therefore the gas is continuously pushed and discharged out of the gas detection main body 32 through the gas outlet through hole 3216a and the gas outlet opening 3261b.

In some embodiments, the gas detection device 3 not only includes the particulate sensor 325 to detect the particulate matters (such as PM₁, PM_{2.5}, and PM₁₀) in the gas to obtain the information of the particulate matters, but also can obtain the property of the gas introduced into the gas detection device 3 (for example, the gas may be formaldehyde, ammonia, carbon monoxide, carbon dioxide, oxygen, ozone, or the like). Therefore, the gas detection device 3 may further include a gas sensor 327. The gas sensor 327 is disposed on the driving circuit board 323 and is located at the gas outlet groove 3216 for detecting the polluted gas introduced into the gas outlet groove 3216, and the gas sensor 327 is electrically connected to the driving circuit board 323. Therefore, the gas sensor 327 can detect the gas contained in the gas outlet path. In some embodiments, the gas sensor 327 includes a volatile organic compound detector capable of detecting information of carbon dioxide or total volatile organic compounds. In some embodiments, the gas sensor 327 includes a formaldehyde sensor capable of detecting information of formaldehyde (HCHO) gas. In some embodiments, the gas sensor 327 includes a bacterial sensor capable of detecting information of bacteria or fungi. In some embodiments, the gas sensor 327 includes a virus sensor capable of detecting information of viruses.

In some embodiments, the central control monitoring device B is one of a central control box and a portable mobile device. The central control box and the portable mobile device receives the outdoor air pollution data and the indoor air pollution data to perform an intelligent computation and comparison on the outdoor air pollution data and the indoor air pollution data, and the central control box and the portable mobile device is configured to transmit the control command intelligently and selectively through the wireless communication. In some embodiments, the central control monitoring device B may be a portable mobile device which is utilized along with an application program of the system for cleaning, zeroing, and preventing air pollution in indoor space to implement the transmission of the control command and to display the outdoor/indoor air pollution data, and the wireless communication may be implemented by using one of a Wi-Fi module, a Bluetooth module, a radiofrequency identification module, and a near field communication module. In this embodiment, the central control monitoring device B may be connected to a cloud device E through network, and the cloud device E receives the outdoor air pollution data and the indoor air pollution data collected by the central control monitoring device B to perform the intelligent computation and comparison on the outdoor air pollution data and the indoor air pollution data to locate the air pollution location in the indoor space, and the cloud device E is configured to transmit the control command intelligently and selectively.

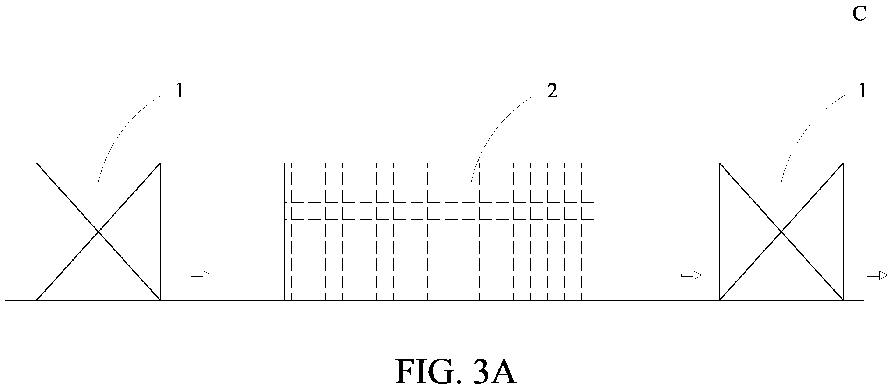

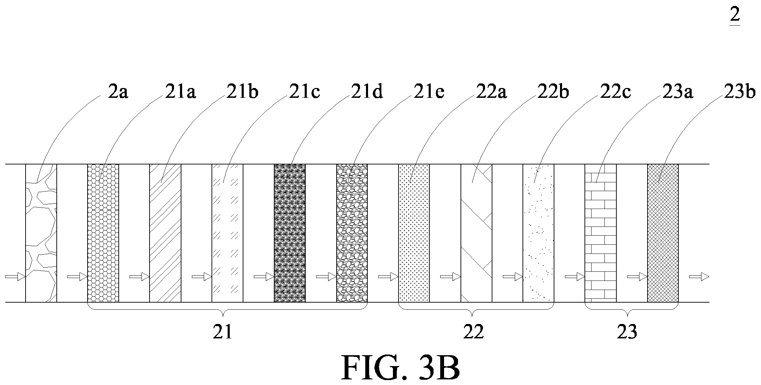

Please refer to Fig. 1A and Fig. 1B. Each of the gas exchange and filtration devices C comprises at least one blower 1 and at least one filtering component 2, wherein the blower 1 has the ability of transmitting the air bi-directionally, including the extraction and ejection. In the present embodiment, the arrow shown in the figures indicates the direction of the air flow. The blower 1 may be disposed in front of the filtering component 2 or behind the filtering component 2, also the blowers 1 may be disposed in front of and behind the filtering component 2 simultaneously (as shown in Fig. 3). Accordingly, the blower 1 can be adjusted and modified according to any practical scenario by the person in the art. Moreover, each of the gas exchange and filtration devices C is combined with a smart switch D. The smart switch D is a unit configured to implement smart switch control of circuitry through the combination and programming of a control board and electronic components; that is, in some embodiments, the smart switch D controls an apparatus to be turned on or off through the wireless communication. The smart switch D receives the control command to enable the operation of the blower 1, and the smart switch D receives the control command through the wireless communication; that is, in some embodiments, the smart switch D receives the control command transmitted by the central control monitoring device B through the wireless communication. In some embodiments, the wireless communication may be implemented by using one of a Wi-Fi module, a Bluetooth module, a radiofrequency identification module, and a near field communication module.

Whether a connection signal of the wireless communication of the smart switch D is normal or abnormal may be determined by the central control monitoring device B, and the central control monitoring device B is capable of displaying or issuing a warning. Therefore, when the connection signal of the wireless communication between one of the gas exchange and filtration devices C and the smart switch D is abnormal, the warning notifies a user to operate the gas exchange and filtration device C manually to achieve an error detection mechanism for preventing failure of communication, thus preventing the operation failure of the system for cleaning, zeroing, and preventing air pollution in indoor space. Hence, by allowing the user to operate the gas exchange and filtration device C manually, the air pollution is filtered and exchanged by the filtering component 2, so that the indoor air pollution data detected by the gas detection devices A in the indoor space approaches to the safety detection value, thus the air pollution in the indoor space is cleaned and the indoor air pollution data approaches to the non-detection state or equals to zero, and the gas in the indoor space is cleaned to the safe and breathable state.

Moreover, as shown in Fig. 1B, in this embodiment, for each of the gas exchange and filtration devices C, the smart switch D is replaced by a gas detection device A disposed in the gas exchange and filtration device C. The gas detection device A controls the gas exchange and filtration device C to be enabled, receives the control command to turn on or turn off the blower 1, and controls airflow adjustment of the blower 1. The gas detection device A disposed in the gas exchange and filtration device C controls the blower 1 to be enabled once the indoor air pollution data exceeds the safety detection value, and the gas detection device A can control airflow adjustment of the blower 1 according to the outdoor air pollution data and the indoor air pollution data, so that the gas exchange and filtration device C can control the operation of the blower 1 in a more smartly. In other words, in this embodiment, when the gas detection device A disposed in the gas exchange and filtration device C detects that the indoor air pollution data exceeds the safety detection value, the gas detection device A actively controls the blower 1 of the gas exchange and filtration device C to be enabled, and the gas detection device A actively controls airflow adjustment of the blower 1 according to the indoor air pollution data, so that the air pollution can be filtered and cleaned by a filtering component 2 nearby the air pollution, thus allowing the filtration and cleaning mechanism of the gas exchange and filtration device C to be performed more smartly, instantly, energy-saving. In some embodiments, the structure of the gas detection device A is identical to the structure of the gas detection device 3, and the control circuit board 31 of the gas detection device A can control the blower 1 to be turned on or turned off and controls airflow adjustment of the blower 1; details for the control and adjustment are not repeated here.

As mentioned, according to one or some embodiments of the present invention, regarding the operation of the system for cleaning, zeroing, and preventing air pollution in indoor space, a plurality of gas detection devices A is configured within an indoor space, and at least one outdoor gas detection device A0 and at least one indoor gas detection A1 are provided to detect and output an outdoor air pollution data and an indoor air pollution data. A central control monitoring device B receives the outdoor air pollution data and the indoor air pollution data through a wireless communication and is connected to a cloud device E. The cloud device performs an intelligent computation and comparison on the outdoor air pollution data and the indoor air pollution data received by the central control monitoring device B. If the indoor air pollution data is greater than the outdoor air pollution data, the cloud device E intelligently transmits a control command, and the central control monitoring device B receives the control command to instantly control the blower 1 of the gas exchange and filtration device C to be enabled, so that the air pollution in the indoor space is rapidly cleaned and discharged to the outdoor space, and the air pollution is circulated rapidly through the filtering component 2 to allow the indoor air pollution data in the indoor space become a safety detection value. In this embodiment, one of the gas exchange and filtration devices C is a heating, ventilation and air conditioning system (HVAC system), and the filtering component 2 is a filter having a minimum efficiency reporting value (MERV) 8 or higher, so that the air pollution in the indoor space is rapidly cleaned and discharged to the outdoor space. In another embodiment, one of the gas exchange and filtration devices C is a fresh air exchange system C1, and the filtering component 2 is a high-efficiency particulate air (HEPA) filter, so that the air pollution in the indoor space is rapidly cleaned and discharged to the outdoor space.

Moreover, the cloud device E performs the intelligent computation and comparison through artificial intelligence (AI) computation and big data comparison on a highest data among the indoor air pollution data to locate the air pollution location in the indoor space. Alternatively or additionally, in some embodiments, the cloud device E performs the intelligent computation and comparison through artificial intelligence (AI) computation and big data comparison on the indoor air pollution data detected by at least three of the indoor gas detection devices A1 to locate the air pollution location in the indoor space through the intelligent computation and comparison. After the cloud device E locates the air pollution location in the indoor space, the cloud device E transmits the control command intelligently and selectively to the central control monitoring device B, and the central control monitoring device B transmits the control command to the gas exchange and filtration devices C to perform the adjusting and controlling mechanism. Under the adjusting and controlling mechanism, the central control monitoring device B transmits the control command to a gas exchange and filtration device C at the air pollution location to enable the gas exchange and filtration device C at the air pollution location firstly to generate an air pollution cleaning path (namely, the blower 1 of the gas exchange and filtration device at the air pollution location is firstly enabled), and then the central control monitoring device B transmits the control command to rest of the gas exchange and filtration devices C which are outside the air pollution location to enable the rest of the gas exchange and filtration devices C (namely, the blowers 1 of the rest of the gas exchange and filtration devices C which are outside the air pollution location are then enabled), and thus an air convection is generated and directed to the air pollution, thus accelerating convection and circulation of the air pollution at the air pollution location. Under the adjusting and controlling mechanism, not only the gas exchange and filtration device C at the air pollution location performs filtration and cleaning of the air pollution at the air pollution location instantly, but also the rest of the gas exchange and filtration devices C which are outside the air pollution location perform filtration and cleaning of the air pollution outside the air pollution location which is diffused, moved, and directed by the air convection, therefore the air pollution in the indoor space is filtered to allow the indoor air pollution data to be the safety detection value in which the air pollution data approaches to the non-detection state or equal to zero, and the gas in the indoor space is cleaned to the safe and breathable state. It should be noted that, in the practical embodiment of the present invention, one of the gas exchange and filtration devices C may comprise at least one selected from the group consisting of a fresh air exchange system C1, a cleaner C2, an air conditioner C3, a ventilator C4, an electric fan C5, and a cooker hood C6, but the present invention is not limited thereto. In other words, the type and the number of the gas exchange and filtration device C are not limited; the system may include one or more gas exchange and filtration devices C. The type and the number of the gas exchange and filtration device C can be adjusted and modified according to the size of the indoor space and the practical requirement for air quality.

Moreover, in one or some embodiments, the filtering component 2 of the gas exchange and filtration device C filters the air pollution, through at least one selected from the group consisting of: by a filter to block and absorb the air pollution to filter the air pollution physically, by applying a degradation layer 21 to filter the air pollution chemically, along with a light illumination 22 to filter the air pollution chemically, and along with a degradation unit 23 to filter the air pollution chemically, but the prevent invention is not limited thereto. In some embodiments, the filter is a high-efficiency particulate air filter 2a for absorbing the chemical smog, bacteria, dusts, particles, and pollens contained in the polluted gas, thereby the polluted gas introduced into the system can be filtered and purified. In some embodiments, the degradation layer 21 may be an activated carbon 21a for filtering organic and inorganic substances and for filtering colored or odor substances. In some embodiments, the degradation layer 21 may be a cleansing factor 21b having chlorine dioxide for suppressing viruses, bacteria, fungus, influenza A virus, influenza B virus, Enterovirus, and Norovirus in the polluted gas introduced into the system. Accordingly, the suppressing rate may exceed 99%, allowing the reduction of the cross infections of the viruses. In some embodiments, the degradation layer 21 may be an herbal protection coating layer 21c including the extracts of *Rhus chinensis* Mill (may be *Rhus chinensis* Mill from Japan) and the extracts of Ginkgo biloba to efficiently perform anti-allergy function and destroy cell surface proteins of influenza viruses (e.g., influenza virus subtype H1N1). In some embodiments, the degradation layer 21 may be a layer of silver ions 21d for suppressing viruses, bacteria, and fungus in the polluted gas introduced into the system. In some embodiments, the degradation layer 21 may be a zeolite mesh 21e for removing ammonia, heavy metals, organic pollutants, Escherichia coli, phenol, chloroform, or anion surfactants. In some embodiments, the light illumination 22 is a photocatalyst unit including a photocatalyst 22a and an ultraviolet light 22b. When the photocatalyst 22a is illuminated by the ultraviolet light 22b, the light energy is converted into electrical energy in order to degrade the hazardous matters in the polluted gas to achieve the effect of filtration and purification. In some embodiments, the light illumination 22 is a photo plasma unit including a nanometer light tube 22c. The introduced polluted gas is illuminated by the nanometer light tube 22c, making the oxygen molecules and water molecules in the polluted gas decompose into photo plasma with high oxidative power for generating a plasma flow which is capable of destroying the organic molecules. Accordingly, volatile organic compounds (VOC) such as formaldehyde and toluene in the polluted gas can be decomposed into water and carbon dioxide. Therefore, the introduced gas can be filtered.

According to one or some embodiments, a system for cleaning, zeroing, and preventing air pollution in indoor space is provided. In the system, a plurality of gas detection devices is configured within an indoor space to detect the qualitative property, the concentration, and the location, a central control monitoring device is configured to receive the outdoor air pollution data and the indoor air pollution data through a wireless communication, and the central control monitoring device is connected to a cloud device to perform mathematical computation and artificial intelligent computation to ensure the location of the air pollution. The central control monitoring device selectively and intelligently transmits a control command to enable a gas exchange and filtration device closest to the air pollution location to generate an air flow to guide the air pollution to be filtered by at least one gas exchange and filtration device, so that the air pollution is filtered and exchanged by the filtering component, until the indoor air pollution data detected by the gas detection devices in the indoor space become the safety detection value, thus the air pollution in the indoor space is cleaned and the indoor air pollution data approaches to the non-detection state or equals to zero, and the gas in the indoor space is cleaned to the safe and breathable state.

## Claims

1. A system for cleaning, zeroing, and preventing air pollution in indoor space, adapted to be implemented in an indoor space, **characterized by** comprising:
a plurality of gas detection devices (A, 3) configured to detect an air pollution in the indoor space, wherein the plurality of gas detection devices (A, 3) comprise at least one outdoor gas detection device (A0) and at least one indoor gas detection device (A1), the at least one outdoor gas detection device (A0) and the at least one indoor gas detection device (A1) are configured to detect a qualitative property and a concentration of the air pollution and respectively output an outdoor air pollution data and an indoor air pollution data;
a central control monitoring device (B), wherein the central control monitoring device (B) is configured to receive the outdoor air pollution data and the indoor air pollution data through a wireless communication to perform an intelligent computation and comparison between the outdoor air pollution data and the indoor air pollution data to locate an air pollution location in the indoor space, and the central control monitoring device (B) is configured to transmit a control command intelligently and selectively through the wireless communication; and
a plurality of gas exchange and filtration devices (C), wherein each of the plurality of gas exchange and filtration devices (C) is combined with a smart switch (D), and the smart switch (D) receives the control command to perform an adjusting and controlling mechanism, so that the air pollution is filtered and exchanged by the plurality of gas exchange and filtration devices (C), thereby allowing the indoor air pollution data detected by the plurality of gas detection devices (A, 3) in the indoor space to be a safety detection value in which the indoor air pollution data approaches to a non-detection state or equals to zero, and the gas in the indoor space is cleaned to a safe and breathable state.

2. The system for cleaning, zeroing, and preventing air pollution in indoor space according to claim 1, wherein the air pollution comprises at least one selected from the group consisting of particulate matters, carbon monoxide, carbon dioxide, ozone, sulfur dioxide, nitrogen dioxide, lead, total volatile organic compounds, formaldehyde, bacteria, fungi, viruses, and any combination thereof.

3. The system for cleaning, zeroing, and preventing air pollution in indoor space according to claim 1, wherein the safety detection value includes at least one selected from the group consisting of a concentration of PM_{2.5} which is less than 15 µg/m³, a concentration of carbon dioxide (CO₂) which is less than 1000 ppm, a concentration of total volatile organic compounds (TVOC) which is less than 0.56 ppm, a concentration of formaldehyde (HCHO) which is less than 0.08 ppm, a colony-forming unit per cubic meter of bacteria which is less than 1500 CFU/m³, a colony-forming unit per cubic meter of fungi which is less than 1000 CFU/m³, a concentration of sulfur dioxide which is less than 0.075 ppm, a concentration of nitrogen dioxide which is less than 0.1 ppm, a concentration of carbon monoxide which is less than 9 ppm, a concentration of ozone which is less than 0.06 ppm, a concentration of lead which is less than 0.15 µg/m³, and any combination thereof.

4. The system for cleaning, zeroing, and preventing air pollution in indoor space according to claim 1, wherein the safety detection value comprises a detection value in which the indoor air pollution data approaches to almost zero or equals to zero.

5. The system for cleaning, zeroing, and preventing air pollution in indoor space according to claim 1, wherein each of the at least one outdoor gas detection device (A0) and the at least one indoor gas detection device (A1) is a gas detection device (3), the gas detection device (3) comprises a control circuit board (31), a gas detection main body (32), a microprocessor (33), and a communication device (34); the gas detection main body (32), the microprocessor (33), and the communication device (34) are integrally packaged and electrically connected to the control circuit board (31); the microprocessor (33) controls the operation of the gas detection main body (32), the gas detection main body (32) detects the air pollution and output a detection signal, and the microprocessor (33) receives the detection signal to perform computation to generate the indoor air pollution data and the outdoor air pollution data and provides the indoor air pollution data and the outdoor air pollution data to the communication device (34) through the wireless transmission so as to transmit the indoor air pollution data and the outdoor air pollution data to the central control monitoring device (B).

6. The system for cleaning, zeroing, and preventing air pollution in indoor space according to claim 5, wherein the gas detection main body (32) comprises:
a base (321), having:
a first surface (3211);
a second surface (3212) opposite to the first surface (3211);
a laser installation region (3213) hollowed out from the first surface (3211) to the second surface (3212);
a gas inlet groove (3214) recessed from the second surface (3212) and located adjacent to the laser installation region (3213), wherein the gas inlet groove (3214) has a gas inlet through hole (3214a) and two lateral walls; two light penetration windows (3214b) penetrate on the two lateral walls of the gas inlet groove (3214) and are in communication with the laser installation region (3213);
a gas-guiding component installation region (3215) recessed from the second surface (3212) and in communication with the gas inlet groove (3214), wherein a ventilation hole (3215a) penetrates a bottom surface of the gas-guiding component installation region (3215); and
a gas outlet groove (3216) including a first region (3216b) and a second region (3216c), wherein the first region (3216b) is corresponding to the gas-guiding component installation region (3215) and is recessed from a portion of the first surface (3211) corresponding to the bottom surface of the gas-guiding component installation region (3215); the second region (3216c) is hollowed out from the first surface (3211) to the second surface (3212) in a region that is not corresponding to the gas-guiding component installation region (3215); the gas outlet groove (3216) is in communication with the ventilation hole (3215a) and has a gas outlet through hole (3216a);
a piezoelectric actuator (322) received in the gas-guiding component installation region (3215);
a driving circuit board (323) covering and attached to the second surface (3212) of the base (321);
a laser component (324) disposed on and electrically connected to the driving circuit board (323), wherein the laser component (324) is received in the laser installation region (3213), and a light path of a light beam emitted by the laser component (324) passes through the light penetration windows (3214b) and is orthogonal to the gas inlet groove (3214);
a particulate sensor (325) disposed on and electrically connected to the driving circuit board (323), wherein the particulate sensor (325) is received in a position of the gas inlet groove (3214) where the light path of the light beam emitted by the laser component (324) is orthogonal to the gas inlet groove (3214), so that particulates in the air pollution passing through the gas inlet groove (3214) which is illuminated by the light beam of the laser component (324) are detected by the particulate sensor (325);
a gas sensor (327) disposed on and electrically connected to the driving circuit board (323), wherein the gas sensor (327) is received in the gas outlet groove (3216) for detecting the air pollution introduced into the gas outlet groove (3216); and
an outer cover (326) covering the base (321) and having a side plate (3261), wherein the side plate (3261) has a gas inlet opening (3261a) and a gas outlet opening (3261b), the gas inlet opening (3261a) is corresponding to the gas inlet through hole (3214a) of the base (321), and the gas outlet opening (3261b) is corresponding to the gas outlet through hole (3216a) of the base (321);
wherein when the outer cover (326) is covered on the base (321) and the driving circuit board (323) is attached to the second surface (3212) of the base (321), a gas inlet path is defined by the gas inlet groove (3214) and a gas outlet path is defined by the gas outlet groove (3216), thereby the piezoelectric actuator (322) is driven to accelerate the introduction of the air pollution outside the gas inlet through hole (3214a) into the gas inlet path defined by the gas inlet groove (3214) from the gas inlet opening (3261a); the air pollution passes through the particulate sensor (325) to detect a particle concentration of the particulates contained in the air pollution; and the air pollution is discharged into the gas outlet path defined by the gas outlet groove (3216) from the ventilation hole (3215a), detected by the gas sensor (327), and discharged out of the gas detection main body (32) from the gas outlet through hole (3216a) and the gas outlet opening (3261b) of the base (321).

7. The system for cleaning, zeroing, and preventing air pollution in indoor space according to claim 6, wherein the particulate sensor (325) is capable of detecting information of particulate matters.

8. The system for cleaning, zeroing, and preventing air pollution in indoor space according to claim 6, wherein the gas sensor (327) comprises at least one selected from the group consisting of a volatile organic compound detector, a formaldehyde sensor, a bacterial sensor, a virus sensor, and any combination thereof, so that the gas sensor (327) is capable of detecting information of carbon dioxide or total volatile organic compounds, information of formaldehyde (HCHO) gas, information of bacteria or fungi, or information of viruses.

9. The system for cleaning, zeroing, and preventing air pollution in indoor space according to claim 1, wherein the central control monitoring device (B) is one of a central control box and a portable mobile device.

10. The system for cleaning, zeroing, and preventing air pollution in indoor space according to claim 1, wherein the central control monitoring device (B) is connected to a cloud device (E) through network, and the cloud device (E) receives the outdoor air pollution data and the indoor air pollution data collected by the central control monitoring device (B) to perform the intelligent computation and comparison on the outdoor air pollution data and the indoor air pollution data to locate the air pollution location in the indoor space, and the cloud device (E) is configured to transmit the control command intelligently and selectively.

11. The system for cleaning, zeroing, and preventing air pollution in indoor space according to claim 10, wherein the cloud device (E) performs the intelligent computation and comparison on the outdoor air pollution data and the indoor air pollution data received by the central control monitoring device (B); if the indoor air pollution data is greater than the outdoor air pollution data, the cloud device (E) intelligently and selectively transmits the control command to the central control monitoring device (B), and according to the control command, the central control monitoring device (B) controls the plurality of gas exchange and filtration devices (C) to be enabled, so that the air pollution in the indoor space is discharged outside the indoor space rapidly, and the air pollution in the indoor space is filtered until the gas in the indoor space is cleaned to the safe and breathable state.

12. The system for cleaning, zeroing, and preventing air pollution in indoor space according to claim 10, wherein the number of the indoor gas detection device (A1) is plural, the cloud device (E) performs the intelligent computation and comparison through artificial intelligence (AI) computation and big data comparison on a highest data among the indoor air pollution data to locate the air pollution location in the indoor space, and the cloud device (E) further performs the intelligent computation and comparison through artificial intelligence (AI) computation and big data comparison on the indoor air pollution data detected by at least three of the indoor gas detection devices (A1) to locate the air pollution location in the indoor space through the intelligent computation and comparison.

13. The system for cleaning, zeroing, and preventing air pollution in indoor space according to claim 12, wherein after the cloud device (E) locates the air pollution location in the indoor space, the cloud device (E) transmits the control command intelligently and selectively to the central control monitoring device (B), and the central control monitoring device (B) transmits the control command to the plurality of gas exchange and filtration devices (C) to perform the adjusting and controlling mechanism; under the adjusting and controlling mechanism, the central control monitoring device (B) transmits the control command to one of the plurality of gas exchange and filtration devices (C) at the air pollution location to enable said one of the plurality of gas exchange and filtration devices (C) at the air pollution location firstly to generate an air pollution cleaning path, and then the central control monitoring device (B) transmits the control command to rest of the plurality of gas exchange and filtration devices (C) which are outside the air pollution location to enable the rest of the plurality of gas exchange and filtration devices (C) to generate an air convection directed to the air pollution, thus accelerating convection and circulation of the air pollution at the air pollution location; under the adjusting and controlling mechanism, not only said one of the plurality of gas exchange and filtration devices (C) at the air pollution location performs filtration and cleaning of the air pollution at the air pollution location instantly, but also the rest of the plurality of gas exchange and filtration devices (C) which are outside the air pollution location perform filtration and cleaning of the air pollution outside the air pollution location which is diffused, moved, and directed by the air convection, and thus the air pollution in the indoor space is filtered to allow the indoor air pollution data to be the safety detection value in which the air pollution data approaches to the non-detection state or equal to zero, and the gas in the indoor space is cleaned to the safe and breathable state.

14. The system for cleaning, zeroing, and preventing air pollution in indoor space according to claim 1, wherein each of the plurality of gas exchange and filtration devices (C) comprises at least one blower (1) and at least one filtrating component (2), the central control monitoring device (B) transmits the control command through the wireless communication, and for each of the plurality of gas exchange and filtration devices (C), the smart switch (D) receives the control command through the wireless communication to control the at least one blower (1) to be enabled; wherein whether the wireless communication of the smart switch (D) is normal or abnormal is determined by the central control monitoring device (B), and the central control monitoring device (B) is capable of displaying or issuing a warning, so that when the wireless communication between one of the plurality of gas exchange and filtration devices (C) and the smart switch (D) is abnormal, the warning notifies a user to operate said one of the plurality of gas exchange and filtration devices (C) manually to achieve an error detection mechanism for preventing failure of communication.

15. The system for cleaning, zeroing, and preventing air pollution in indoor space according to claim 14, wherein the smart switch (D) for each of the plurality of gas exchange and filtration devices (C) is replaced by a gas detection device (A), the gas detection device (A) controls respective one of the plurality of gas exchange and filtration devices (C) to be enabled, receives the control command to turn on or turn off the at least one blower (1), and controls airflow adjustment of the at least one blower (1).
